# FASCICULE DE BREVET EUROPEEN

(11) **EP 1 358 354 B1**
(45) Date de publication et mention de la délivrance du brevet: **29.03.2006**
(21) Numéro de dépôt: 02701394.5
(22) Date de dépôt: 04.02.2002
(51) Int. Cl.: C12Q 1/68, A61P 3/06

(54) **PROCEDES D'IDENTIFICATION DE COMPOSES MODULANT LE TRANSPORT INVERSE DU CHOLESTEROL**
VERFAHREN ZUR IDENTIFIZIERUNG VON MODULATOREN DES CHOLESTERINRÜCKWÄRTSTRANSPORTES
METHOD FOR IDENTIFYING COMPOUNDS MODULATING REVERSE CHOLESTEROL TRANSPORT

(30) Priorité: 05.02.2001 FR 0101486
(43) Date de publication de la demande: 05.11.2003
(73) Titulaire: Genfit, 59120 Loos (FR)
(72) Inventeur: STAELS, Bart, B-7850 Petit Enghien (BE)
(74) Mandataire: Tezier Herman, Béatrice
(86) Numéro de dépôt international: PCT/FR2002/000410
(87) Numéro de publication internationale: WO 2002/063038

(56) Documents cités:
- WO-A-00/40965
- WO-A-00/57915
- DE-A- 19 709 125
- US-A- 5 721 096
- LAFFITTE B A ET AL: "Identification of the DNA binding specificity and potential target genes for the farnesoid X-activated receptor." JOURNAL OF BIOLOGICAL CHEMISTRY, vol. 275, no. 14, 7 avril 2000 (2000-04-07), pages 10638-10647, XP002181057
- REPA J J ET AL: "Regulation of absorption and ABC1-mediated efflux of cholesterol by RXR heterodimers." SCIENCE, vol. 289, no. 5484, 2000, pages 1524-1529, XP000952750
- PATENT ABSTRACTS OF JAPAN vol. 2000, no. 07, 29 septembre 2000 (2000-09-29) & JP 2000 109455 A (SHIONOGI & CO LTD), 18 avril 2000 (2000-04-18)
- PARKS D J ET AL: "Bile acids: Natural ligands for an orphan nuclear receptor." SCIENCE, vol. 284, no. 5418, 21 mai 1999 (1999-05-21), pages 1365-1368, XP000892009
- CLAUDEL T ET AL: "Bile acids suppress human apolipoprotein A-I gene expression via a negative response element for the nuclear receptor FXR." CIRCULATION, vol. 104, no. 17 Supplement, 23 octobre 2001 (2001-10-23), page II.291 XP008004251 Scientific Sessions 2001 of the American Heart Association; Anaheim, CA, USA; 11-14 November 2001 ISSN: 0009-7322
- CLAUDEL T ET AL: "Bile acid-activated nuclear receptor FXR suppresses apolipoprotein A-I transcription via a negative FXR response element." THE JOURNAL OF CLINICAL INVESTIGATION, vol. 109, no. 7, avril 2002 (2002-04), pages 961-971, XP002201491 ISSN: 0021-9738

## Description

La présente invention concerne des méthodes et composés susceptibles de moduler le transport inverse du cholestérol chez un mammifère ainsi que des méthodes de criblage permettant de sélectionner, d'identifier et/ou de caractériser des composés capables de moduler le transport inverse du cholestérol. Elle concerne également des cellules, vecteurs et constructions génétiques utilisables pour la mise en oeuvre de ces méthodes, ainsi que des compositions pharmaceutiques destinées au traitement de l'athérosclérose.

L'athérosclérose est une cause majeure de morbidité, de mortalité, d'infarctus du myocarde, d'ischémie cérébrale, de maladies cardiovasculaire et de la vascularisation périphérique. L'hypercholestérolémie et la surcharge en cholestérol des macrophages, impliquée dans l'inflammation vasculaire, sont des facteurs majeurs qui contribuent à l'athérosclérose. L'hypercholestérolémie est actuellement traitée grâce à la combinaison d'un régime alimentaire et d'une intervention médicamenteuse avec, par exemple les statines ou des agents séquestrant les acides biliaires. Le développement de nouvelles stratégies thérapeutiques est cependant nécessaire pour pallier aux limites des thérapies existantes.

Le transport inverse du cholestérol, mis en oeuvre par les HDL (« High Density Lipoproteins » ou Lipoprotéines de Haute Densité), permet de décharger le cholestérol qui s'accumule dans les tissus périphériques et assure son élimination métabolique via le foie. Il contribue ainsi à la protection de l'organisme contre l'athérosclérose. L'apolipoprotéine A-I (apo AI) est un constituant fondamental des HDL, responsable de leur efficacité. A cet égard, l'augmentation de l'expression de l'apo AI a un effet protecteur contre l'athérosclérose. L'expression de l'apo AI est régulée par des hormones ou des agents thérapeutiques tels que les fibrates. Le rôle crucial des récepteurs nucléaires tels que HNF4, PPARa ou RORα dans le contrôle de la transcription du gène apo AI a été démontré. PPARα est notamment responsable de l'augmentation de l'expression de Papa AI par les fibrates observée chez l'homme et utilisée en clinique humaine pour le traitement des dislipidémies. L'identification de nouvelles voies de signalisation intraccllulaire impliquées dans le contrôle de l'expression de l'apo AI permettrait donc de définir de nouvelles stratégies thérapeutiques susceptibles d'augmenter l'efficacité du transport inverse du cholestérol et donc de protéger contre l'athérosclérose.

Les récepteurs nucléaires aux hormones forment une grande famille de facteurs de transcription dont l'activité est modulable par des ligands naturels et/ou artificiels. Ces facteurs de transcription contrôlent l'expression de leurs gènes-cibles en se liant généralement à des éléments de réponse spécifiques agissant en cis et en recrutant des protéines accessoires nécessaires à l'activation de la machinerie transcriptionnelle.
Le récepteur FXR, également connu comme RIP14 ou NR1H4, a été initialement identifié comme étant le récepteur aux farnesoïdes. Il agit en formant un hétérodimère avec le récepteur RXR, également membre de cette famille de protéines (cf. Lafitte B A et al. : Journal of Biological Chemistry. 2000, 275(14) : 10638-10647). US-A-5 721 096 décrit des méthodes de criblage de composés capables d'activer l'expression du gène codant pour l'apo AI et faisant intervenir des cellules hépatiques modifiées par une séquence promotrice du gène de l'apoAl, à laquelle se lient des protéines nucléaires, et en particulier le récepteur RXRα complexé à la protéine ARP-I. Récemment, il a été démontré que plusieurs acides biliaires tels que l'acide chénodéoxycholique (CDCA : chenodeoxycholic acid) ou l'acide déoxycholique (DCA: deoxycholic acid) ainsi que, dans une moindre mesure, l'acide lithocholique (LCA : lithocholic acid), sont les ligands véritables de FXR et que ces composés activent le récepteur FXR.
FXR se lie préférentiellement sur un élément de réponse constitué d'une répétition inversée de deux motifs AGGTCA séparés d'un nucléotide. Il se lie également à des répétitions du motif AGGTCA séparées de 2, 4 ou 5 nucléotides (DR-2, DR-4, DR-5) (Laffitte B A et al., J. Biol. Chem.. 275: 10638-10647. (2000)). Plusieurs gènes cibles de FXR ont été identifiés tels que Cyp7α-hydroxylase (voir WO 00/40965), I-BABP. PI,TP ou CPT-II.

La présente invention est fondée sur l'observation du rôle de FXR dans l'expression du gène humain codant pour l'apo AI ainsi que sur l'interaction directe qui se produit entre FXR et deux fragments distincts du promoteur de ce gène. Elle est fondée également sur l'observation originale d'une répression de l'activité du promoteur de l'apo AI humain, par la sur-expression de FXR en présence d'acides biliaires. Elle se fonde également sur l'identification des sites C et A du promoteur du gène de l'apo AI humaine comme étant des éléments de réponse fonctionnels à FXR et la caractérisation de leur séquence respective. Elle se fonde également sur l'observation originale que FXR lié au site C réprime l'expression de l'apo AI, que FXR se fixe également sur ce site de façon inattendue et imprévisible comme monomère et que la répression de l'activité du promoteur du gène de l'apo AI humaine par FXR via le site C est dominante par rapport à son action d'activation via le site A.

La présente invention démontre ainsi pour la première fois une modulation de la production de l'apo AI par le récepteur nucléaire FXR. La présente invention fournit ainsi de nouvelles cibles et de nouvelles approches pour la recherche de composés capables de réguler l'expression de cette protéine, ou l'activité des HDL, ou le transport inverse du cholestérol.

Selon un mode de réalisation particulier, les méthodes de criblage selon l'invention incluent plus particulièrement les étapes suivantes :
- la mise en contact d'un ou de plusieurs composés avec une construction d'acide nucléique comprenant au moins un élément de réponse à FXR du promoteur du gène humain de l'apo AI ou un variant fonctionnel de celui-ci, dans des conditions susceptibles de permettre aux dits composés de se fixer sur ledit élément de réponse,
- la détermination de la liaison éventuelle desdits composés sur le ou les élément(s) de réponse, et
- éventuellement la comparaison de la mesure précédente avec une mesure réalisée dans les mêmes conditions mais avec une construction d'acide nucléique comprenant au moins une copie mutée d'un élément de réponse à FXR du promoteur de l'apo AI humaine.
Selon une forme particulière de réalisation du procédé de l'invention, les conditions susceptibles de permettre aux dits composés de se fixer sur le ou lesdits élément(s) de réponse à FXR comprennent la présence du récepteur FXR (par exemple sous forme de monomère) ou d'un équivalent fonctionnel, et la détermination de l'effet de la présence du composé test sur la liaison de FXR à l'élément de réponse.

Selon un autre mode de réalisation particulier (test d'activité transcriptionnelle), on mesure l'effet d'un ou de plusieurs composés tests sur l'activité transcriptionnelle d'un promoteur comprenant au moins un élément de réponse à FXR selon l'invention. Un tel test est préférentiellement réalisé en système cellulaire, par détermination de l'expression d'un gène rapporteur placé sous le contrôle d'un tel promoteur, notamment dans une cellule comprenant (e.g., exprimant, de manière naturelle ou recombinante) le récepteur FXR ou un équivalent fonctionnel de celui-ci.

Une forme préférée de mise en oeuvre de l'invention consiste à utiliser une cassette d'expression combinant un ou plusieurs éléments de réponse à FXR, selon l'invention, avec un gène rapporteur. Avantageusement, ledit gène rapporteur est placé sous le contrôle d'un promoteur comprenant au moins une copie du ou desdits éléments de réponse, par exemple, le promoteur de l'apo AI ou des variants ou fragments de celui-ci. Tout gène connu de l'homme du métier dont l'activité ou la présence dans des extraits biologiques est facilement mesurable peut être utilisé comme gène rapporteur pour la mise en oeuvre du procédé de criblage.

Les composés susceptibles d'être identifiés par le procédé de l'invention peuvent être des composés de nature, structure et origine variées, notamment des composés biologiques, des facteurs nucléaires, des cofacteurs, etc., des composés chimiques, synthétiques, etc., capables de modifier l'activité de FXR. Il peut s'agir également de banques, notamment de chimiothèques ou de banques de protéines, peptides ou acides nucléiques, par exemple de clones codant des protéines ou peptides liant l'ADN.

Les méthodes selon l'invention peuvent être utilisées pour sélectionner, identifier ou caractériser des composés capables de modifier la liaison de FXR à l'un et/ou l'autre de ses élément(s) de réponse et/ou de moduler (i.e. augmenter ou diminuer) l'expression du gène codant pour l'apo AI humaine et/ou de moduler l'activité des HDL et/ou de moduler le transport inverse du cholestérol.

Dans le but de faciliter la compréhension de la présente demande, les définitions suivantes sont fournies, qui précisent ou complètent leur signification habituelle.

« Apolipoprotéine AI » ou apo AI : L'apolipoprotein A-I est une protéine de 243 acides aminés qui contient une extrémité amino-terminale globulaire et une extrémité carboxy-terminale qui est capable de se lier aux lipides (Segrest et al, Cur. Op. Lipidol., 11:105-115 (2000)). Cette protéine est un constituant majeur des lipoprotéines de haute densité et joue un rôle fondamental dans le transport inverse du cholestérol (Fruchart et Duriez, Biochimie, 80:167-172 (1998), Leroy et al., Cur. Op. Lipidol., 6:281-285 (1995)). Le gène, l'ADNc et l'ARNm de l'apo AI ont été clonés et séquencés (Breslow et al., PNAS 79:6861-6865 (1982), Shoulders et Baralle, Nuc. Ac. Res., 10:4873-4882 (1982), Karathanasis et al., Nature 304:371-373 (1983)), et sont accessibles sur banques de données Genbank® (par exemple sur Internet à l'adresse : http://www.ncbi.nlm.nih-gov) sous les numéros d'accès : NM_000039, M20656 (Promoteur) et J00098).

"High Density Lipoproteine (HDL)": Les particules HDL sont des lipoprotéines de haute densité (1,063-1,21g/ml) qui sont réputées pour avoir un rôle protecteur contre l'athérosclérose principalement du fait de leur capacité à extraire le cholestérol des cellules périphériques et à promouvoir son retour vers le foie où il est éliminé (Fruchart et Duriez, Biochimie, 80:167-172 (1998)). L'apo AI est le constituant protéique majeur des HDL, représentant jusqu'à 70% des protéines. Elles comportent également de l'apo AII, de l'apo CI, de l'apo CII, de l'apo CIII et de l'apo E en plus faible proportion.

"FXR" : Le récepteur FXR a été isolé, caractérisé et séquencé chez l'homme et chez la souris (Forman et al., Cell, 81 :687-693 (1995), Seol et al., Mol. Endo, 9 :72-85 (1995)). La séquence de l'ARNm est disponible également sur banques de données Genbank®, sous les numéros d'accès NM_005123, NM_021745 et U18374. La région de FXR impliquée dans la liaison à l'ADN (« DNA Binding Domain ») est principalement comprise entre les résidus Cys 124-Met 189 de la protéine humaine (correspondant à 726-953 dans NM_005123) ou entre les résidus 423-621 (U18374) de la protéine de souris.

L'expression « équivalent fonctionnel » qui fait référence au récepteur FXR, désigne tout polypeptide dérivé de la structure du récepteur FXR et conservant la capacité de liaison à l'élément de réponse notamment tout élément de réponse des séquences SEQ ID NO : 1 ou 2 ou des variants fonctionnels de celles-ci. Les équivalents fonctionnels peuvent être des variants naturels (polymorphisme, épissage, etc.), des fragments, mutants, délétants, etc.. Préférentiellement, il s'agit de polypeptides comprenant au moins une région d'acides aminés identique à 60% au moins à celle du récepteur FXR, de manière préférentielle à 75% au moins et de manière encore plus préférentielle à 90-95% au moins. L'expression inclut également les fragments du récepteur FXR, notamment les fragments contenant le site de liaison à l'ADN du récepteur FXR.

Le terme "transport inverse" est employé pour désigner le mécanisme physiologique, parfois défaillant, par lequel le cholestérol en excès dans les tissus périphériques est pris en charge par des lipoprotéines de haute densité, les HDL (High Density Lipoprotein), puis transporté vers le foie où il est éliminé.

### A. Identification d'un élément de réponse à FXR.

La présente invention démontre l'implication et le mécanisme d'action de FXR dans la régulation de l'expression de l'apo AI par les acides biliaires et, ce faisant, dans la régulation du transport inverse du cholestérol. En présence d'acides biliaires, la sur-expression de FXR se traduit par une diminution de l'activité de ce dernier. L'invention révèle, par ailleurs, la séquence précise de deux éléments de réponse à FXR, au sein du promoteur du gène codant pour l'apo AI humaine. L'invention porte également sur des constructions particulières, notamment des acides nucléiques comprenant des éléments de réponse à FXR, ainsi que des cassettes, vecteurs et cellules recombinantes les contenant.

Ainsi l'invention fournit les séquences (SEQ ID NO : 1 et SEQ ID NO : 2) des deux éléments de réponse à FXR, identifiés initialement au sein du promoteur du gène humain de l'apo AI, responsables d'une interaction entre FXR et le promoteur apo AI et de la régulation par FXR de l'expression de l'apo AI.

Ainsi, la présence de trois copies du site C (SEQ ID NO : 1) provoque une inhibition par FXR et les acides biliaires de l'expression d'un gène rapporteur (cf.: exemple 5). A l'inverse, la présence de trois copies du site A (SEQ ID NO : 2) provoque une stimulation de ladite expression (cf. : exemple 5).
Un objet particulier de l'invention réside dans un acide nucléique comprenant la séquence SEQ ID NO : 1 ou 2, ou un variant fonctionnel de celle-ci (« élément de réponse FXR »).
Un autre objet de l'invention réside dans une construction d'acide nucléique comprenant un élément de réponse FXR tel que défini ci-dessus. Il peut s'agir notamment d'une cassette d'expression comprenant au moins une copie d'un élément de réponse tel que défini ci-avant.
L'invention concerne encore tout promoteur artificiel ou chimérique comprenant un élément de réponse à FXR tel que défini ci-avant.
Les variants fonctionnels de l'élément de réponse selon l'invention, peuvent être tout dérivé ou fragment de la séquence native conservant la capacité de lier le récepteur FXR. Généralement, les variants conservent au moins 50% des résidus de la séquence native décrite dans la présente demande. Classiquement, les variants possèdent des modifications affectant moins de 5 nucléotides dans la séquence considérée. Préférentiellement, il s'agit d'un séquence identique à 60% au moins, de manière préférentielle à 75% au moins et de manière encore plus préférentielle à 90% au moins à la séquence native décrite dans la présente demande.
Les variants peuvent comporter différents types de modifications tels qu'une ou plusieurs mutations ponctuelles ou non, additions, délétions et/ou substitutions.
Ces modifications peuvent être introduites par les méthodes classiques physiques, chimiques ou de la biologie moléculaire, telles que notamment la mutagenèse dirigée ou, plus pratiquement, par synthèse artificielle de la séquence dans un synthétiseur.
Les variants peuvent être testés pour leur capacité de liaison à FXR de différentes façons, et notamment :
(i) par mise en contact de la séquence test avec le récepteur FXR (par exemple dans un test acellulaire), et détection de la formation d'un complexe (par exemple par retard de migration sur gel) ;
(ii) par insertion de la séquence test dans une cassette d'expression comprenant un promoteur minimal et un gène rapporteur, introduction de la cassette dans une cellule, et détection (le cas échéant dosage) de l'expression du gène rapporteur en présence et en l'absence de FXR ;
(iii) par toute autre technique connue de l'homme du métier, permettant de mettre en évidence l'interaction entre un acide nucléique et une protéine, par exemple.

L'invention a encore pour objet des variants inactifs des éléments de réponse définis ci-dessus, notamment des variants essentiellement incapables de lier le récepteur FXR. Des exemples de tels variants sont notamment les séquences SEQ ID NO: 3 et 4.
Ces variants inactifs peuvent être préparés et testés dans les conditions décrites ci-dessus pour les variants fonctionnels.
Les variants selon l'invention possèdent avantageusement la capacité d'hybrider avec la séquence SEQ ID NO : 1 ou 2 ou une partie de celle-ci.

### B. Méthodes de sélection, d'identification et de caractérisation de composés qui modulent le transport inverse du cholestérol.

L'invention décrit des méthodes d'identification de composés qui modulent (i.e., augmentent ou diminuent) le transport inverse du cholestérol. Ces composés peuvent agir en altérant la liaison de FXR à son ou ses ligands (acides biliaires, corépresseurs et çoactivateurs, etc.). Ils peuvent encore modifier, voir supprimer, la liaison de FXR seul ou de FXR et de ses cofacteurs, à son ou ses élément(s) de réponse et ainsi modifier l'expression du gène humain de l'apo AI. La fixation de FXR à l'élément de réponse C (SEQ ID NO : 1) diminue ainsi la transcription du gène humain de l'apo AI et réduit le transport inverse du cholestérol. L'utilisation de composés capables d'inhiber la fixation de FXR à cet élément de réponse, où FXR joue le rôle de répresseur permet donc au contraire d'augmenter la transcription du gène humain de l'apo AI et de stimuler le transport inverse du cholestérol.
La présente invention démontre également de façon surprenante que la fixation de FXR à l'élément de réponse A (SEQ ID NO : 2) augmente la transcription du gène humain de l'apo AI et augmente le transport inverse du cholestérol. L'utilisation de composés capables de stimuler la fixation de FXR à cet élément de réponse, où FXR joue cette fois le rôle d'activateur de la transcription (ou de reproduire cette activation par fixation directe), permet donc également d'augmenter la transcription du gène humain de l'apo AI et de stimuler le transport inverse du cholestérol.
La présente invention décrit ainsi de nouvelles méthodes pour la sélection, l'identification ou la caractérisation de composés capables d'augmenter le transport inverse du cholestérol.

### 1. Méthodes basées sur un criblage d'expression.

La présente invention concerne une méthode pour la sélection, l'identification ou la caractérisation de composés capables de moduler le transport inverse du cholestérol, qui comprend :
- la mise en contact d'un composé test avec une cellule hôte comprenant une cassette d'expression d'un gène rapporteur, ladite cassette comprenant un gène rapporteur placé sous le contrôle d'un promoteur contenant au moins une copie d'un élément de réponse à FXR du promoteur du gène humain de l'apo AI ou d'un variant fonctionnel de celui-ci, et
- la détermination de l'expression du gène rapporteur.

Les méthodes selon l'invention, prévoient plus spécifiquement la mise en contact d'un composé test avec une construction d'acide nucléique ou une cassette d'expression contenant au moins une copie d'un élément de réponse à FXR (SEQ ID NO : 1 ou 2). Selon un autre mode de réalisation de l'invention, la copie du ou des élément(s) de réponse à FXR (site C et/ou A) peut être une copie mutée, ladite copie mutée étant essentiellement incapable de lier le récepteur FXR, même en présence d'acides biliaires.
Selon un mode d'exécution particulier des méthodes de l'invention, il est en outre prévu de comparer les effets éventuels, déterminés grâce à l'une de ces méthodes avec ceux, éventuels, déterminés grâce à une méthode réalisée dans les mêmes conditions mais avec une construction d'acide nucléique comprenant au moins un variant inactif (par exemple, une copie mutée) d'un élément de réponse à FXR du promoteur du gène codant pour l'apo AI humain (SEQ ID NO : 1 ou 2) ou d'un variant fonctionnel de ce dernier.

Les procédés de l'invention peuvent être mis en oeuvre avec différents types de cellules, de promoteur, de gènes rapporteurs, et dans différentes conditions, comme il est décrit ci-après.

### a) Mise en contact des composés avec la cellule hôte

Certaines méthodes de criblage, décrites par l'invention, prévoient ainsi une étape de mise en contact du composé test avec des cellules hôtes, dans des conditions particulières qui permettent de déterminer l'expression dans lesdites cellules d'un gène rapporteur et d'obtenir ainsi une information concernant l'effet du composé test. Classiquement, l'effet du composé test est comparé au niveau d'expression du gène rapporteur déterminé en l'absence dudit composé (et/ou avec un élément de réponse muté).
Ces cellules, dans un mode préféré de l'invention, peuvent être des cellules de mammifères (hépatocytes, fibroblastes, cellules endothéliales, musculaires, etc.).
De manière encore plus préférée, ces cellules peuvent être des cellules humaines. Il peut également s'agir de cultures primaires ou de lignées établies. Dans un autre mode de mise en oeuvre, il est possible également d'utiliser des cellules procaryotes (bactéries), des cellules de levure (Saccharomyces, Kluyveromyces, etc.), des cellules végétales, etc..
Les composés peuvent être mis au contact des cellules à différents moments, selon leur(s) effet(s), leur concentration, la nature des cellules et l'appréciation technique.
Le contact peut être effectué sur tout support approprié et notamment sur une plaque, dans un tube ou une flasque. Généralement, la mise en contact est réalisée dans une plaque multipuits ce qui permet de conduire, en parallèle, des essais nombreux et variés. Parmi les supports typiques on trouve des plaques de microtitration et plus particulièrement des plaques 96 ou 384 puits (ou plus), faciles à manipuler et sur lesquels la révélation peut être obtenue grâce à une stimulation classique.
Selon le support et la nature du composé test, des quantités variables de cellules peuvent être utilisées lors de la mise en oeuvre des méthodes décrites. De manière classique, 10³ à 10⁶ cellules sont mises en contact avec un type de composé test, dans un milieu de culture approprié, et de manière préférentielle entre 10⁴ et 10⁵ cellules. A titre d'exemples, dans une plaque de 96 puits, 10⁵ cellules peuvent être incubées dans chaque puit avec une quantité voulue d'un composé test. Dans une plaque de 384 puits, moins de 10⁵ cellules et typiquement entre 1x10⁴ et 4x10⁴ cellules sont généralement incubées dans chaque puit avec le composé test.
La quantité (ou la concentration) de composé test, peut être ajustée par l'utilisateur selon le type de composé (sa toxicité, sa capacité de pénétration cellulaire, etc.), le nombre de cellules, la longueur de la période d'incubation, etc.. Généralement, les cellules sont exposées à des quantités de composés test qui varient de 1nM à 1mM. Il est bien sûr possible de tester d'autres concentrations sans dévier de la présente invention. Chaque composé peut de plus être testé en parallèle à différentes concentrations.
Différents adjuvants et/ou vecteurs et/ou produits facilitant la pénétration des composés dans les cellules tels que des liposomes, des lipides cationiques, des polymères, de la pénétratine, du Tat PDT, des peptides issus d'adénovirus (penton ou fibres) ou d'autres virus, etc. peuvent en outre être utilisés si nécessaire.
Le contact est maintenu entre 5 et 72 heures, généralement entre 12 et 48 heures. En effet, les cellules et les divers réactifs doivent de préférence rester en contact suffisamment longtemps pour permettre la synthèse de novo du produit d'expression du gène rapporteur. De manière préférée, l'incubation dure environ 36 heures.

### b) Détermination de l'activité des composés.

La méthode proposée par l'invention pour sélectionner, identifier ou caractériser des composés capables de moduler le transport inverse du cholestérol prévoit la transformation des cellules hôtes avec une cassette d'expression d'un gène rapporteur. Ledit gène rapporteur peut être notamment tout gène dont le produit de transcription ou d'expression peut être détecté ou dosé dans des extraits biologiques. Il peut s'agir, par exemple, du gène codant pour l'apo AI humaine lui-même, ou encore du gène codant pour la lucifèrase et plus particulièrement pour la lucifèrase de luciole ou pour celle de Renilla, pour la phosphatase alcaline sécrétée, la galactosidase, la lactamase, la Chloramphenicol acetyl transférase (CAT), l'hormone de croissance humaine (hGH), la β-glucuronidase (Gluc) et la Green fluorescent protein (GFP) etc.. Il est entendu que le terme « gène » désigne, au sens large, tout acide nucléique, notamment un ADNc, un ADNg, un ADN synthétique, un ARN, etc..
Le gène rapporteur, quel qu'il soit, est placé sous le contrôle d'un promoteur comprenant au moins une copie d'un élément de réponse à FXR tel que défini ci-avant.
Le gène rapporteur peut donc être placé sous le contrôle de tout promoteur dont la séquence comprend l'une des séquences SEQ ID NO : 1 et/ou 2 ou un variant fonctionnel de celles-ci. Cette séquence particulière peut être présente à raison d'une ou de plusieurs copies dans le promoteur (préférentiellement 1 à 10 et encore plus préférentiellement 1 à 6), en amont, en aval ou en interne, dans la même orientation ou dans l'orientation opposée. Dans un mode préféré de mise en oeuvre de l'invention, le gène rapporteur est placé sous le contrôle d'un promoteur qui comprend une ou plusieurs copies du site A (SEQ ID NO : 2) et aucun site C. Dans un autre mode de mise en oeuvre de l'invention, le gène rapporteur est placé sous le contrôle d'un promoteur qui comprend une ou plusieurs copies du site C (SEQ ID NO : 1) et aucun site A. Dans un autre mode de mise en oeuvre de l'invention, le gène rapporteur est placé sous le contrôle d'un promoteur qui comprend une ou plusieurs copies des sites C et A. Préférentiellement, il s'agit d'un promoteur dont le différentiel d'activité en l'absence et en présence de FXR ou d'un équivalent fonctionnel peut être détecté.
Pour la réalisation d'un promoteur de l'invention, l'élément de réponse à FXR peut être associé à un promoteur minimal transcriptionnel. Le promoteur minimal est un promoteur transcriptionnel ayant une activité basale faible ou inexistante, et susceptible d'être augmentée en présence d'un activateur transcriptionnel (l'interaction de FXR en présence d'acides biliaires avec le site A). Un promoteur minimal peut donc être un promoteur naturellement faible dans les cellules de mammifère, c'est-à-dire produisant une expression non toxique et/ou non suffisante pour obtenir un effet biologique prononcé. Avantageusement, un promoteur minimal est une construction préparée à partir d'un promoteur natif, par délétion de région(s) non essentielle(s) à l'activité transcriptionnelle. Ainsi, il s'agit de préférence d'un promoteur comprenant essentiellement une boîte TATA, généralement d'une taille inférieure à 160 nucléotides, centrée autour du codon d'initiation de la transcription. Un promoteur minimal peut ainsi être préparé à partir de promoteurs viraux, cellulaires, forts ou faibles, tels que par exemple le promoteur du gène de la thymidine kinase (TK) du virus de l'herpès, le promoteur inunédiat du CMV, le promoteur PGK, le promoteur SV40, etc.. Le promoteur minimal peut avoir une activité suffisamment élevée pour permettre d'identifier des composés qui augmentent l'inhibition par FXR, via le site C ou augmentent l'activation par FXR, via le site A, par exemple.
Le promoteur (P), l'élément de réponse à FXR (FR) et le gène rapporteur (GR) sont agencés de manière fonctionnelle dans la cassette d'expression, c'est-à-dire de sorte que le promoteur minimal contrôle l'expression dudit gène et que son activité soit régulée par FXR. Généralement, ces régions sont donc disposées dans l'ordre suivant, dans l'orientation 5'->3' : ER-P-GR. Toutefois, tout autre agencement fonctionnel peut être envisagé par l'homme du métier sans départir de la présente invention.
En outre, les différents domaines fonctionnels ci-dessus peuvent être liés directement les uns aux autres, ou séparés par des nucléotides n'affectant pas significativement le caractère fonctionnel de la cassette d'expression ou permettant de conférer des caractéristiques ou performances améliorées au système (amplificateur, silencer, intron, site d'épissage, etc.).

La méthode de sélection, d'identification et de caractérisation de composés capables de moduler le transport inverse du cholestérol prévoit une étape de détermination de l'expression du gène rapporteur. Il peut s'agir d'une détermination de l'activité transcriptionnelle. A cette fin, l'ARN total est extrait des cellules en culture dans des conditions expérimentales d'une part et dans une situation témoin d'autre part. Cet ARN est utilisé comme sonde pour analyser, par exemple, les changements dans l'expression du ou des gène(s) rapporteur(s).
Il peut également s'agir d'une révélation de l'expression du gène rapporteur à l'aide d'un substrat adapté. Cette révélation peut être obtenue à l'aide de techniques variées dont la nature dépend du type de gène rapporteur utilisé. La mesure peut, par exemple, correspondre à une densité optique ou à une émission fluorescente dans le cas d'une utilisation comme gène rapporteur du gène codant pour la β-galactosidase ou la luciférase.
Dans un mode particulier, l'expression du gène rapporteur est mesurée à travers le niveau d'hydrolyse d'un substrat du produit d'expression du gène rapporteur. Par exemple, de nombreux substrats peuvent être utilisés pour évaluer l'expression de la β-lactamase. Il peut notamment s'agir de tout produit contenant un noyau β-lactame et dont l'hydrolyse peut être contrôlée. Les substrats préférés sont ceux spécifiques de la β-lactamase (i.e., ils ne sont généralement pas hydrolysés dans les cellules de mammifères en l'absence de β-lactamase), ceux qui ne sont pas toxiques à l'égard des cellules de mammifères et/ou dont le produit d'hydrolyse peut être contrôlé facilement, par exemple par des méthodes basées sur la fluorescence, la radioactivité, une activité enzymatique ou toute autre méthode de détection.
Des substrats encore plus préférés sont les substrats ratiométriques. L'hydrolyse de ces substrats peut être directement reliée à l'activité du produit d'expression du gène rapporteur par le nombre de cellules. Un substrat ratiométrique spécifique et non toxique utilisable dans la présente invention est le CCF2-AM.
La concentration du substrat peut être ajustée par l'homme du métier en fonction du nombre de cellules, par exemple. Les cellules sont généralement maintenues en contact avec le substrat pendant environ 60 minutes.
La présence du produit du gène rapporteur (ou du produit d'hydrolyse du substrat) peut être déterminée par des méthodes classiques connues de l'homme du métier (fluorescence, radio..., D.O., luminescence, FRET (voir WO 0037077), SPA, biopuces, méthodes immunologiques, etc.).
Généralement, on détermine l'activité d'un composé test dans une cellule et cet effet est comparé au niveau d'activité en l'absence de composé test ou à une valeur moyenne déterminée en l'absence de tout composé test.
La mesure de l'hydrolyse implique essentiellement une mesure (ou la détermination de la quantité relative) du produit d'hydrolyse contenu dans chaque échantillon réactionnel. Cette mesure peut être réalisée grâce à différentes techniques connues de l'homme du métier, incluant la détection d'une fluorescence, d'une radioactivité, d'une couleur, d'une activité enzymatique, d'un immun complexe antigène. anticorps, etc.. De manière préférée, le produit d'hydrolyse, est détecté et quantifié grâce à une technique de détection de fluorescence. Des fluorochromes variés peuvent ainsi être utilisés et contrôlés sur des échantillons de cellules.
Un test secondaire permettant de valider, chez l'animal, la sélection des composés, peut aussi être réalisé grâce à la détermination de la quantité d'HDL exprimées ou grâce à la détermination d'une variation significative du transport inverse du cholestérol aux niveau de cellules traitées avec lesdits composés par comparaison avec des cellules non traitées. Il est également possible de mesurer le taux plasmatique de cholestérol et/ou de déterminer l'expression hépatique de l'apo AI.

D'une manière préférentielle, on utilise dans la méthode selon l'invention, une cellule hôte comprenant le récepteur FXR ou un équivalent fonctionnel.
La présence du récepteur FXR permet de reproduire une situation physiologique et permet d'identifier, grâce aux méthodes précédemment décrites, des composés capables de moduler les interactions entre FXR et l'un et/ou l'autre de ses élément(s) de réponse, tel(s) que divulgué(s) par la présente invention, ou entre FXR et un ou plusieurs ligand(s) de FXR.
Le récepteur FXR peut être naturellement présent ou peut avoir été introduit ou ajouté artificiellement. Il peut s'agir d'un équivalent de FXR à savoir toute séquence d'acides aminés identique à 60% au moins à celle du récepteur FXR, de manière préférentielle à 75% au moins et de manière encore plus préférentielle à 90-95% au moins.

Selon un mode préféré, l'invention décrit également une méthode pour la sélection, l'identification ou la caractérisation de composés capables de moduler le transport inverse du cholestérol, qui comprend :
■ la mise en contact d'un composé test avec une cellule hôte comprenant :
   - une cassette d'expression d'un gène rapporteur, ladite cassette comprenant un gène rapporteur placé sous le contrôle d'un promoteur contenant au moins une copie d'un élément de réponse à FXR du promoteur du gène humain de l'apo AI ou d'un variant fonctionnel de celui-ci, et
   - le récepteur FXR ou un équivalent fonctionnel, et
■ la détermination de l'expression du gène rapporteur.

Cette méthode permet de déterminer le niveau d'expression du gène rapporteur, selon l'une des techniques connues de l'homme du métier décrites précédemment, en présence du composé test ou en l'absence dudit composé, une augmentation ou une diminution du niveau d'expression du gène rapporteur signalant l'aptitude du composé test à moduler le transport inverse du cholestérol.
Selon un mode encore plus préféré de l'invention, la cellule hôte comprend également un ligand de FXR.
La désignation « ligand de FXR » s'applique non seulement aux acides biliaires et dérivés mais également aux facteurs de transcription, aux co-activateurs et corépresseurs, ainsi qu'aux autres polypeptides impliqués dans la machinerie de régulation de l'expression de gènes. Il peut s'agir, par exemple, d'autres récepteurs comme RXR ou les récepteurs aux hormones nucléaires.

Comme indiqué précédemment, ces méthodes permettent le criblage, rapide et en parallèle, de nombreux composés test sur une ou plusieurs populations cellulaires (cellules de mammifère, cellules humaines telle que, par exemple, des hépatocytes, cellules procaryotes, etc.). Ces méthodes sont prédictives, automatisables et adaptées à la sélection, l'identification et la caractérisation desdits composés.
Une forme particulière de mise en oeuvre du procédé de criblage utilise les méthodes classiques d'identification de clones qui expriment des protéines qui lient l'ADN. Il peut s'agir par exemple de cribler des banques d'expression d'ADNc dans λgt11 ou d'utiliser la méthode dite du «One Hybrid » ou du « Phage Display », ou encore de pratiquer une purification par chromatographie d'affinité. La ou les protéine(s) isolée(s) sont ensuite séquencées.

### 2. Méthodes basées sur un test de liaison.

L'invention concerne également une méthode pour la sélection, l'identification ou la caractérisation de composés capables de moduler le transport inverse du cholestérol, basée sur la mesure de la liaison d'un composé test à l'un ou l'autre voire aux deux éléments de réponse. Cette méthode comprend plus particulièrement :
- la mise en contact d'un composé test avec une construction d'acide nucléique comprenant au moins une copie d'un élément de réponse à FXR du promoteur du gène humain de l'apo AI ou d'un variant fonctionnel de celui-ci, et
- la détermination de la liaison éventuelle dudit composé test sur l'élément de réponse.

La liaison du composé test sur l'un au moins des éléments de réponse à FXR peut être mise en évidence grâce à une migration sur gel, par électrophorèse des hétérodimères formés suite à la mise en oeuvre de la méthode décrite ci-dessus. Certains composés tests sont effectivement susceptibles de porter un site de liaison à l'ADN en grande partie identique à celui de FXR et d'exercer ainsi une compétition avec celui-ci.
L'électrophorèse permet de distinguer directement les hétérodimères FXR/élément de réponse à FXR, des hétérodimères composé test/élément de réponse à FXR et des éléments de réponse à FXR.
D'autres méthodes basées sur la luminescence ou utilisant la technique FRET (Fluorescence Resonance Energy Transfer) bien connue de l'homme du métier ou la technique SPA (Scintillation Proximity Assay), peuvent être mises en oeuvre dans le cadre de la présente invention pour déterminer la liaison éventuelle du composé test sur l'un et/ou l'autre élément(s) de réponse à FXR.

Cette méthode de sélection, d'identification et de caractérisation directe de composés capables de moduler le transport inverse du cholestérol peut, selon un mode particulier de réalisation de la présente invention, être effectuée en présence du récepteur FXR ou d'un équivalent fonctionnel de ce dernier. L'étape ultérieure de cette méthode consiste alors à déterminer l'effet de la présence du composé test sur la liaison de FXR à son et/ou ses élément(s) de réponse. Il s'agit, par exemple, d'établir la capacité dudit composé test à moduler la liaison de FXR à l'élément de réponse, en déterminant la quantité de FXR liée en présence du composé test par rapport à cette quantité en l'absence de composé test. Un test de compétition utilisant la technique FP (fluorescence Polarization) connue de l'homme du métier peut ainsi s'appliquer efficacement dans le cadre de cette détermination.
Un composé test capable de moduler la liaison de FXR à l'élément de réponse pourra faire l'objet d'un test ultérieur de sa capacité à moduler l'expression d'un gène rapporteur et/ou le transport inverse du cholestérol, selon l'une des méthodes décrites précédemment.

Dans un mode particulier, la construction d'acide nucléique comprend au moins 1 copie, de préférence de 2 à 5 copies de la séquence SEQ ID NO :1 ou d'un variant fonctionnel de celle-ci. Les composés tests capables d'inhiber (c'est-à-dire de réduire au moins partiellement) la liaison de FXR sur cette construction permettent d'activer l'expression du gène rapporteur et constituent des candidats pour stimuler le transport inverse du cholestérol.

### C. Activité des HDL et de l'apolipoprotéine AI.

Les méthodes décrites précédemment pour la sélection, l'identification ou la caractérisation de composés capables de moduler l'expression d'un gène rapporteur et/ou le transport inverse du cholestérol peuvent, selon un autre mode de réalisation de l'invention, être utilisées pour la sélection, l'identification ou la caractérisation de composés capables de moduler l'activité des HDL et/ou l'expression de l'apo AI.

### D. Composés test.

La présente invention peut être appliquée à tout type de composé test. Ainsi, le composé test peut être tout produit qui se présente sous une forme isolée ou en mélange avec d'autres produits. Le composé peut être défini en termes de structure et/ou de composition ou ne pas être défini. Le composé peut, par exemple, être un produit isolé et structurellement défini, un produit isolé de structure indéfinie, un mélange de produits connus et caractérisés ou une composition indéfinie comprenant un ou plusieurs produits. De telles compositions indéfinies peuvent être, par exemple, des échantillons de tissus, des fluides biologiques, des surnageants cellulaires, des préparations végétales, etc.. Les composés test peuvent être des produits inorganiques ou organiques et notamment un polypeptide (ou une protéine ou un peptide), un acide nucléique, un lipide, un polysaccharide, un composé chimique ou biologique tel qu'un facteur nucléaire, un cofacteur ou tout mélange ou dérivé de ces derniers. Le composé peut être d'origine naturelle ou synthétique et inclure une banque combinatoire, un clone ou une banque de clones d'acides nucléiques exprimant un ou plusieurs polypeptide(s) liant l'ADN, etc..

La présente invention est particulièrement adaptée à la sélection, l'identification ou la caractérisation d'un nombre important de composés. Ce criblage simple et efficace peut être accompli en un laps de temps très court. Les méthodes décrites peuvent en particulier être partiellement automatisées, autorisant ainsi le criblage efficace et simultané de composés divers et nombreux, soit sous forme de mélange soit sous forme séparée.

### E. Utilisation des composés identifiés.

Les composés identifiés selon l'invention présentent des propriétés avantageuses pour une utilisation thérapeutique, notamment dans le domaine de l'athérosclérose. Une application possible de l'invention est de permettre l'identification d'une substance thérapeutique susceptible de réduire l'effet inhibiteur des acides biliaires sur l'expression de l'apo AI et d'augmenter ainsi le transport inverse du cholestérol. L'invention permet ainsi l'utilisation d'un composé capable de moduler (i.e., augmenter ou diminuer) la liaison de FXR aux éléments de réponse du promoteur du gène codant pour l'apo AI humain ou d'un variant fonctionnel de celui-ci, pour la préparation d'une composition destinée à moduler (i.e., augmenter ou diminuer) le transport inverse du cholestérol.

Cette utilisation peut être destinée à moduler (i.e., augmenter ou diminuer) l'activité des HDL ou à moduler l'expression de l'apo AI.
Une utilisation d'un composé capable de contrôler l'effet de FXR sur la transcription du gène humain de l'apo AI ou d'un variant fonctionnel de celui-ci peut être destinée à accroître le transport inverse du cholestérol.
Il peut s'agir d'un composé chimique
ou d'un composé biologique. Il peut s'agir d'un facteur nucléaire ou d'un cofacteur. Selon un mode encore plus préféré, il s'agit d'un clone exprimant un ou plusieurs polypeptide(s) liant l'ADN. D'une manière générale, il peut s'agir de tout composé sélectionné, identifié ou caractérisé selon l'une des méthodes précédemment décrites.

L'invention permet l'utilisation de tout composé (ou dérivés desdits composés) sélectionné, identifié ou caractérisé selon l'une des méthodes précédemment décrites, dans le cadre de la présente invention, comme cible de recherches expérimentales ou pour la fabrication de compositions pharmaceutiques destinées à augmenter le transport inverse du cholestérol ou à traiter l'hypercholestérolémie, l'athérosclérose, les désordres lipidiques et/ou les affections cardio-vasculaires.

D'autres avantages et applications de la présente invention apparaîtront à la lecture des exemples qui suivent, qui doivent être considérés comme purement illustratifs et non limitatifs.

### LÉGENDES DES FIGURES

- Figuré n°1 :: Le TCA réduit le taux plasmatique de cholestérol et d'apo AI ainsi que l'expression hépatique de l'apo AI dans les souris transgéniques pour le gène humain de l'apo AI.
- Figure n°2 :: Le CDCA et le TCA réduisent l'expression de l'apo AI dans les cellules HepG2.
- Figure n°3 :: Effet transcriptionnel des acides biliaires.

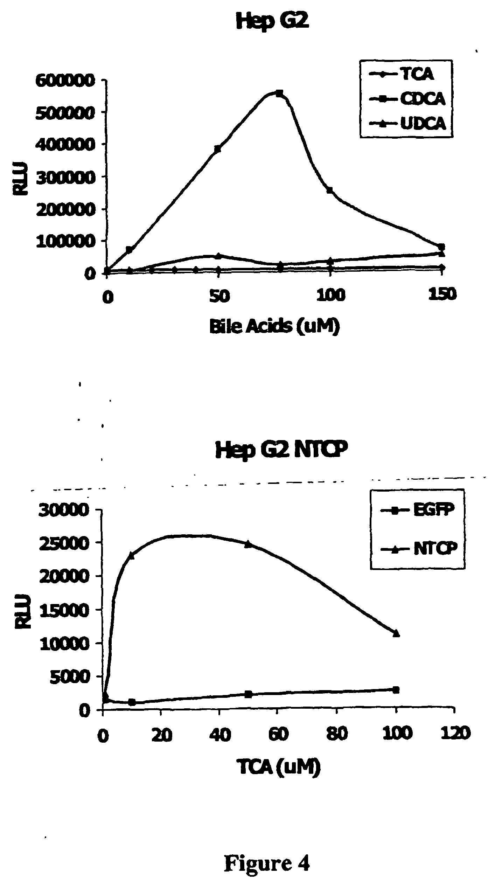

- Figure n°4 :: Le CDCA et le TCA activent FXR.
- Figure n°5 :: La sur-expression de FXR en présence de CDCA réduit l'activité du promoteur de l'apo AI humaine dans les cellules HepG2.
- Figure n°6 :: La sur-expression de FXR en présence de CDCA réduit l'activité du promoteur de l'apo AI humaine dans les cellules HepG2 : identification fonctionnelle des éléments de réponse.
- Figure n°7 :: FXR se lie sur les sites C (SEQ ID NO : 1) et A (SEQ ID NO : 2) du promoteur de l'apo AI en dimère avec RXR alors que FXR se lie également en monomère sur le site C (SEQ ID NO : 1).
- Figure n°8 :: Le GW4064, activateur spécifique de FXR, réduit l'expression de l'apo AI humaine ainsi que l'activité de son promoteur dans les cellules HepG2

### SÉQUENCES

SEQ ID NO : 1 (Site C wt)
SEQ ID NO : 2 (Site A wt)
SEQ ID NO : 3 (Site C mt)
SEQ ID NO : 4 (Site A mt)
SEQ ID NO : 5 (Promoteur de l'apo AI - j04066 (apoAI gene) 1819-2167)
SEQ ID NO : 6 (promoteur Tk - M80483 (pBLCAT5) 38-204; J02224 (Herpes simplex) 302-462)

### EXEMPLES

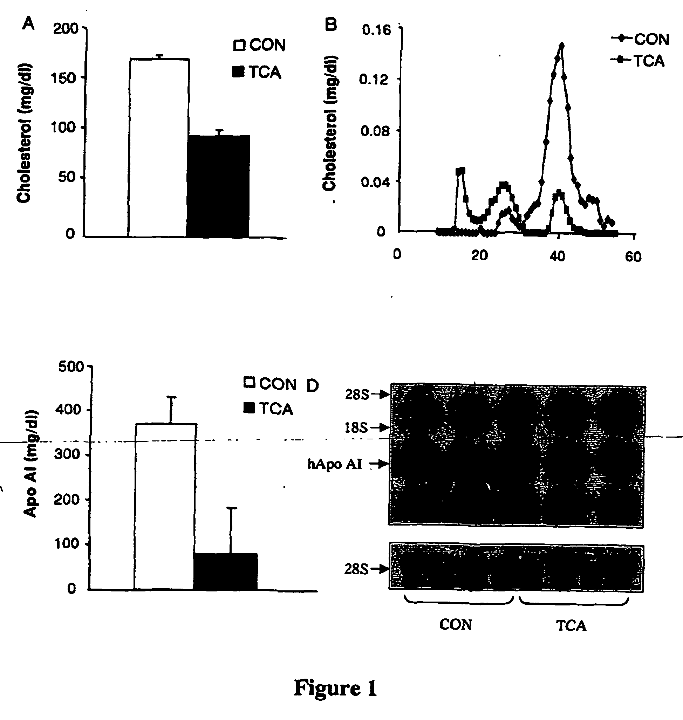

### Exemple 1 : Effet du TCA sur le taux plasmatique de cholestérol et d'apo AI ainsi que sur l'expression hépatique de l'apo AI dans les souris transgéniques pour le gène humain de l'apo AI (figure n°1)

L'exemple 1 montre l'effet d'un régime enrichi en taurocholate sur le taux sérique de cholestérol et d'apo AI humaine ainsi que sur le profil lipidique et l'expression hépatique de l'apo AI humaine de souris transgéniques qui expriment le gène de l'apo AI dans un fond génétique C57BL.
Dix souris transgéniques qui expriment le gène de l'apo AI dans un fond génétique C57BL (IFFA-CREDO, L'Arbresle, France) sont génotypées par PCR et divisées en deux groupes de cinq animaux. Le premier groupe reçoit un régime normal pendant une semaine alors que l'autre est traité par un régime enrichi en TCA (acide taurocholique) (0,5% P/P). Le sang est collecté après un jeûne de 12 heures par ponction retro-orbitale sous anesthésie au barbital. Les échantillons de sérum collectés sont conservés à -20°C avant analyse. Après une semaine de traitement, les animaux sont sacrifiés et des échantillons de foie prélevés et congelés pour analyse. Les taux sériques d'apo AI humaine sont mesurés avant et après le régime comme décrit précédemment (Peters, J. Biol. Chem., 272 (43) : 27307-12 (1997)). Les taux sériques de cholestérol et les profils lipidiques après FPLC sont mesurés comme établis précédemment (Raspé et al., J. Lipid Res., 40 (11) : 2099-2110 (1999)). Les ARNm totaux sont extraits des échantillons de foie comme décrit précédemment (Chomczynski et al., Anal. Biochem., 162 (1) : 156-9 (1987)) et quantifiés par hybridation de Northern Blot (Peters, 1997) à l'aide des sondes apo AI humaine et 28S (Peters, J. Biol. Chem., 272 (43) : 27307-12 (1997)).

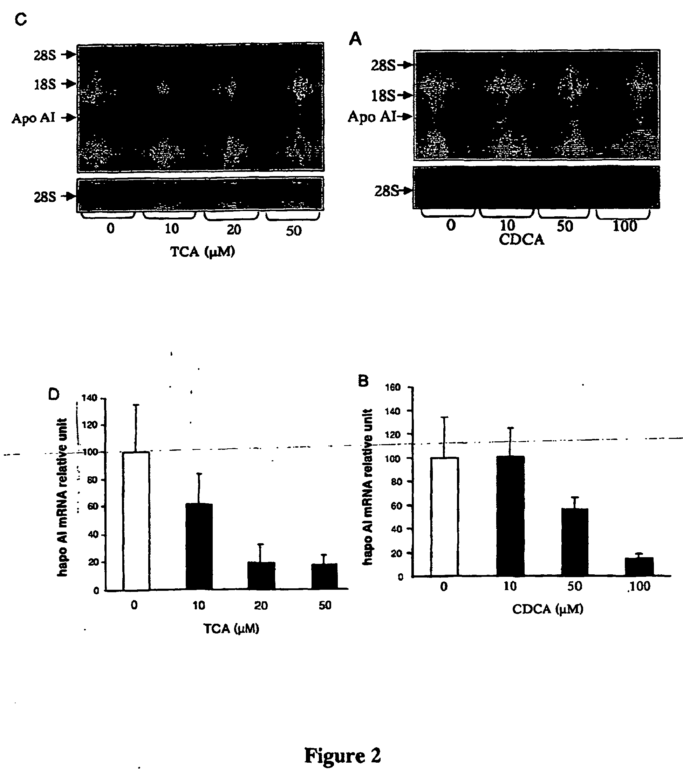

### Exemple 2 : Effet du CDCA et du TCA sur l'expression de l'apo AI dans les cellules HePG2 (figure n°2)

L'exemple 2 montre les effets de concentrations croissantes du CDCA et du TCA qui diminuent l'expression de l'apo AI dans les cellules HepG2. Les cellules HepG2 sont cultivées dans du milieu DMEM supplémenté de 10% de sérum de veau foetal, de penicillin/streptomycin, de pyruvate de sodium et d'acides aminés non-essentiels et incubées à 37°C, dans une atmosphère humide contenant 5% CO₂/95% air. Elles sont ensuite traitées avec les concentrations indiquées de CDCA. Les ARNm totaux sont extraits comme décrit précédemment (Chomczynski et al., Anal. Biochem., 162 (1) : 156-9 (1987)) et quantifiés par hybridation de Northern Blot (Peters, J. Biol. Chem., 272 (43) : 27307-12 (1997)) à l'aide des sondes apo AI humaine et 28S (Peters, J. Biol. Chem., 272 (43) : 27307-12 (1997)).

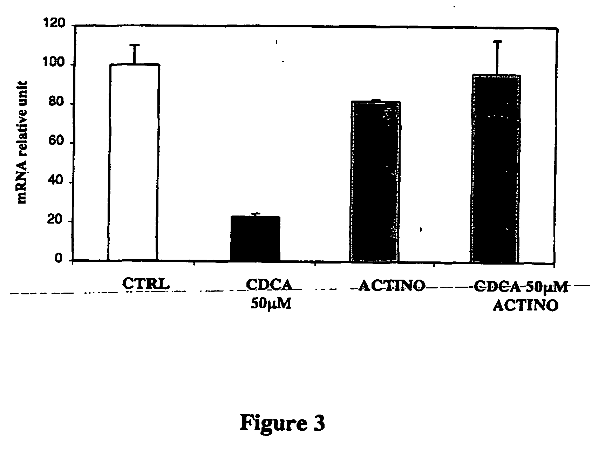

### Exemple 3 : Démonstration d'un effet transcriptionnel des acides biliaires (figure n°3)

L'exemple 3 montre que le CDCA testé à une concentration de 50 µM réduit considérablement l'expression de l'apo AI dans les cellules HepG2. Son effet est compensé par une préincubation de 12h en présence d'actinomycine D (5g/ml), ce qui suggère que l'effet est transcriptionnel. Les cellules HepG2 sont cultivées dans du milieu DMEM supplémenté de 10% de sérum de veau foetal, de penieillin/streptomycin, de pyruvate de sodium et d'acides aminés non-essentiels et incubées à 37°C, dans une atmosphère humide contenant 5% CO₂/95% air. Les ARNm totaux sont extraits comme décrit précédemment (Chomczynski et al., Anal. Biochem., 162 (1) : 156-9 (1987)), et quantifiés par hybridation de Northern Blot (Peters, J. Biol. Chem., 272 (43) : 27307-12 (1997)) à l'aide des sondes apo AI humaine et 28S (Peters, J. Biol. Chem., 272 (43) : 27307-12 (1997)).

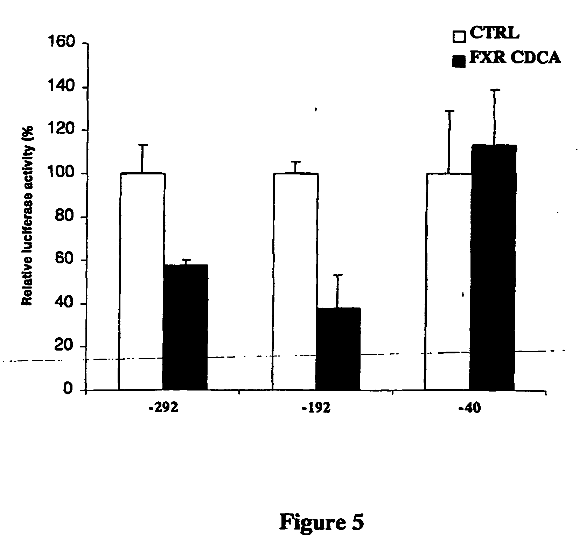

### Exemple 4: Effet de la sur-expression de FXR en présence de CDCA sur l'activité du promoteur de l'apo AI humaine dans les cellules HepG2 (figure n°5)

L'exemple 4 montre que la sur-expression de hFXR et mRXRα en présence de CDCA réprime l'activité des fragments -254/+91 (qui comporte les sites AB et C), [(voir SEQ ID NO : 5)], -192/+91 (qui comporte les site B et C) mais pas du fragment -40/+91 (qui ne comporte que le promoteur minimum) du promoteur de l'apo AI, clonés en amont du gène rapporteur luciférase.
Des cellules HepG2 sont co-transfectées par la technique de lipofection avec 300 ng du vecteur pCDNA3-hFXR qui permet la sur-expression de FXR, 300 ng du vecteur pSG5-mRXRα et 1 µg des vecteurs rapporteurs indiqués qui permettent l'expression du gène rapporteur luciférase sous le contrôle des fragments -254/+91, -192/+91 et -40/+91 du promoteur de l'apo AI. Ces constructions sont obtenues par l'échange du gène rapporteur CAT des constructions décrites précédemment (Ngoc Vu-Dac et al., J. Biol. Chem., 269 (49): 31012-8 (1994)) avec le gène rapporteur Luciférase extrait du plasmide pGL3 de Promega (Madison, WI, USA) comme décrit précédemment (Raspé et al., J. Lipid Res., 40 (11) : 2099-2110 (1999)). La quantité totale d'ADN est établie à 2 µg à l'aide du plasmide pBKS+. Après 3 heures de transfection, les cellules sont incubées dans le milieux de culture avec les acides biliaires, aux concentrations indiquées, pendant 36 heures. L'activité Luciférase est ensuite mesurée comme décrit précédemment (Raspé et al., J. Lipid Res., 40 (11) : 2099-2110 (1999)).

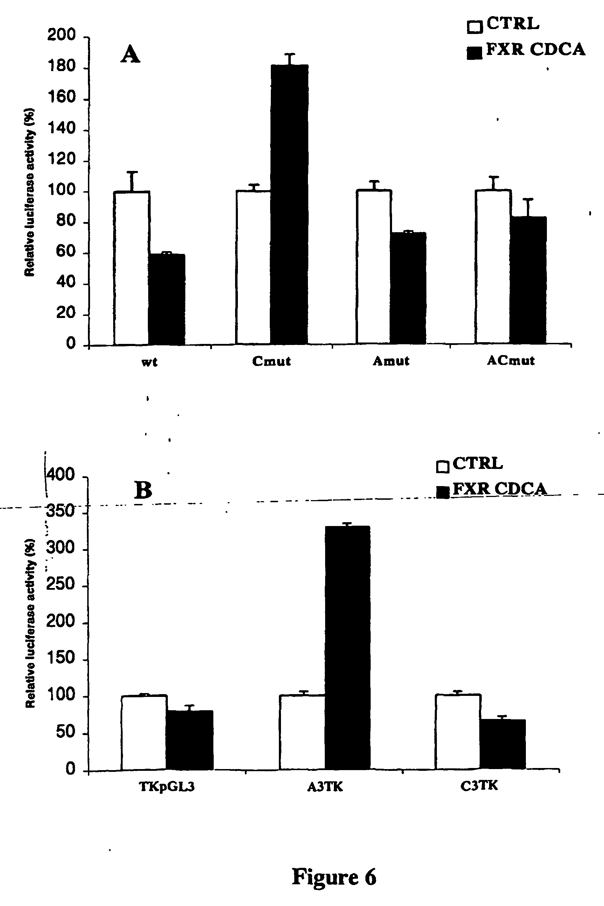

### Exemple 5: Identification fonctionnelle des éléments de réponse à FXR présents sur le promoteur du gène humain de l'apo AI (figure n°6)

L'exemple 5 montre que la sur-expression de hFXR en présence de CDCA réprime l'activité du fragment -254/+91 sauvage du promoteur de l'apo AI (construction notée wt), clonés en amont du gène rapporteur luciférase (panneau A). Il montre également que la mutation du site C présent dans le fragment -254/+91 du promoteur de l'apo AI (construction notée C mut), clonés en amont du gène rapporteur luciférase se traduit par une activation de l'activité transcriptionnelle de la construction en réponse à la sur-expression de hFXR (panneau A). La sur-expression de hFXR en présence de CDCA réprime également l'activité du fragment 254/+91 du promoteur de fapo AI, clonés en amont du gène rapporteur luciférase dont le site A est muté (construction notée A mut) (panneau A). Enfin, la mutation conjointe des site A et C (construction notée AC mut) du fragment -254/+91 du promoteur de l'apo AI clonés en amont du gène rapporteur luciférase se traduit par une perte de la réponse à hFXR.
L'exemple 5 montre que la sur-expression de hFXR en présence de CDCA réprime l'activité d'une construction qui comporte trois copies du site C du promoteur de l'apo AI humaine clonées en amont du promoteur de la thymidine kinase du virus de l'Herpes simplex et du gène rapporteur luciférase (notée C3TK) alors qu'une construction qui comporte trois copies du site A du promoteur de l'apo AI humaine clonées en amont du promoteur de la thymidine kinase du virus de l'Herpès simplex (notée A3TK) est activée par la sur-expression de hFXR en présence de CDCA et que l'activité d'une construction qui ne comporte que le promoteur de la thymidine kinase du virus de l'Herpes simplex (notée TkpGL3) n'est pas affectée par la sur-expression de hFXR en présence de CDCA (panneau B).

Les cellules HepG2 sont co-transfectées par la technique de lipofection avec 300 ng du vecteur pCDNA3-hFXR qui permet la sur-expression de ce dernier et 1 µg des vecteurs rapporteur indiqués qui permettent l'expression du gène rapporteur luciférase sous le contrôle du fragment -254/+91 sauvage ou muté du promoteur du gène humain de l'apo AI, sous le contrôle du promoteur de la thymidine kinase du virus de l'Herpes simplex ou sous le contrôle de trois copies du site A ou du site C du gène humain de l'apo AI clonées en amont du promoteur de la thymidine kinase du virus de l'Herpes simplex. La construction qui comporte le fragment -254/+91 sauvage du promoteur de l'apo AI est obtenue par l'échange du gène rapporteur CAT de la construction correspondante décrite précédemment (Ngoc Vu-Dac et al., J. Biol. Chem., 269 (49) : 31012-8 (1994))) avec le gène rapporteur Luciférase extrait du plasmide pGL3 de Promega (Madison, WI, USA), comme décrit précédemment (Raspé et al., J. Lipid Res., 40 (11) : 2099-2110 (1999)). La construction qui comporte le fragment -254/+91 du promoteur de l'apo AI dont le site A est muté est obtenue par mutagenèse dirigée du site A, dans la construction sauvage, à l'aide du kit Quick Change Site directed mutagenesis (Stratagene, La Jolla, CA, USA), de l'oligonucléotide 5'-CCCCACTGAACCCTTGATTCCTGCTTTGCAGCC-3' (SEQ ID NO: 4) et de l'oligonucléotide complémentaire. La construction qui comporte le fragment -254/+91 du promoteur de l'apo AI dont le site C est muté est obtenue par mutagenèse dirigée du site C, dans la construction sauvage, à l'aide du kit Quick Change Site directed mutagenesis (Stratagene, La Jolla, CA, USA), de l'oligonucléotide 5'-CAGAGCTATATATTATATATATAAGTTCC-3' (SEQ ID NO: 3) et de l'oligonucléotide complémentaire. La construction qui comporte le fragment -254/+91 du promoteur de l'apo AI dont les sites A et C sont mutés est obtenue par mutagenèse dirigée du site A, dans la construction Cmut, à l'aide du kit Quick Change Site directed mutagenesis (Stratagene, La Jolla, CA, USA), de foligonucléotide 5'-CCCCACTGAACCCTTGATTCCTGCTZTGCAGCC-3' (SEQ ID NO: 4) et de l'oligonucléotide complémentaire. La construction qui comporte le promoteur de la thymidine kinase du virus de l'Herpes simplex cloné en amont du gène rapporteur luciférase notée TkpGL3 a été décrit précédemment (Raspé et al., J. Biol. Chem., 276 (4) : 2865-2871 (2001)). La construction notée C3TK qui comporte trois copies du site C du promoteur de l'apo AI humaine clonées en amont du promoteur de la thymidine kinase du virus de l'Herpès simplex et du gène rapporteur luciférase a été obtenue par clonage orienté de trois copies de l'oligonucléotide double brin correspondant à la séquence du site C sauvage (SEQ ID NO : 1) dans le site Smal du vecteur TkpGL3. La construction notée A3TK qui comporte trois copies du site A du promoteur de l'apo AI humaine clonées en amont du promoteur de la thymidine kinase du virus de l'Herpes simplex et du gène rapporteur luciférase a été obtenue par clonage orienté de trois copies de l'oligonucléotide double brin correspondant à la séquence du site A sauvage (SEQ ID NO : 2) dans le site SmaI du vecteur TkpGL3. La quantité totale d'ADN est établie à 2 µg à l'aide du plasmide pBKS+. Après 3 heures de transfection, les cellules sont incubées dans le milieux de culture avec les acides biliaires, aux concentrations indiquées, pendant 36 heures. L'activité Luciférase est ensuite mesurée comme décrit précédemment (Raspé et al., J. Lipid Res., 40 (11) : 2099-2110 (1999)).

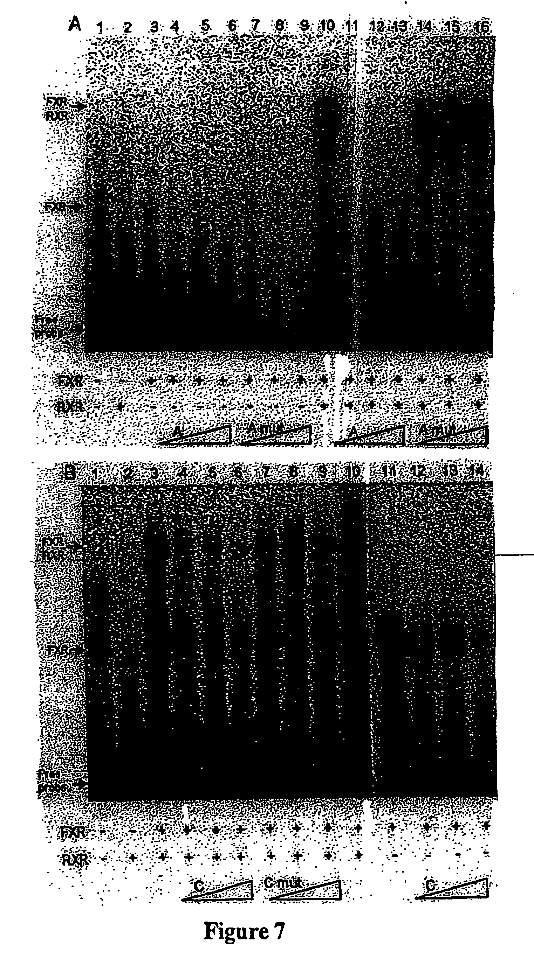

### Exemple 6: Liaison du complexe FXR/RXR au site A (SEQ ID NO:2) et de FXR et du complexe FXR/RXR au site C (SEQ ID NO : 1) du promoteur de l'apo AI (figure n°7)

L'exemple 6 montre que le complexe RXRα/hFXR se lie spécifiquement sur les sites A et C du promoteur de l'apo AI humaine. Il montre également que FXR est capable de se lier seul en monomère uniquement au site C du promoteur de l'apo AI humaine.

Les protéines hFXR et mRXRα sont produites in vitro à l'aide du kit TNT-T7 de Promega (Madison, WI, USA) et des vecteurs pCDNA3-hFXR et pSG5-mRXRα. Des oligonucléotides doubles brins correspondants aux sites A et C du promoteur du gène humain de l'apo AI (SEQ ID NO: 2, SEQ ID NO : 1) et préparés comme décrit précédemment (Ngoc Vu-Dac et al., J. Biol. Chem., 269 (49) : 31012-8 (1994)), ont été marqués avec du [γ-³²P]-ATP à l'aide de la polynucléotide kinase. 2 µl de lysat de réticulocytes programmés par hFXR et mRXRα sont incubés pendant 15 minutes à température ambiante dans un volume final de 20µl de tampon qui contient 10 mM HEPES, 2,5 mM MgCl₂, 10% glycérol, 2,5 mg/ml BSA, 50 mM NaCl et 0,5 mM DTT avec 2,5 µg de polydI-dC et 1 µg d'ADN de sperme de hareng avant l'addition de la sonde marquée (0,5 ng). Le mélange est incubé pendant 15 min. à température ambiante et les complexes sont séparés par électrophorèse sur gel non dénaturant dans du tampon TBE 0,25X. Les expériences de super-retard sont réalisées en ajoutant l'anticorps anti-FXR (Santa Cruz, Californie, USA) 3 heures avant l'addition de l'ADN à 4°C.

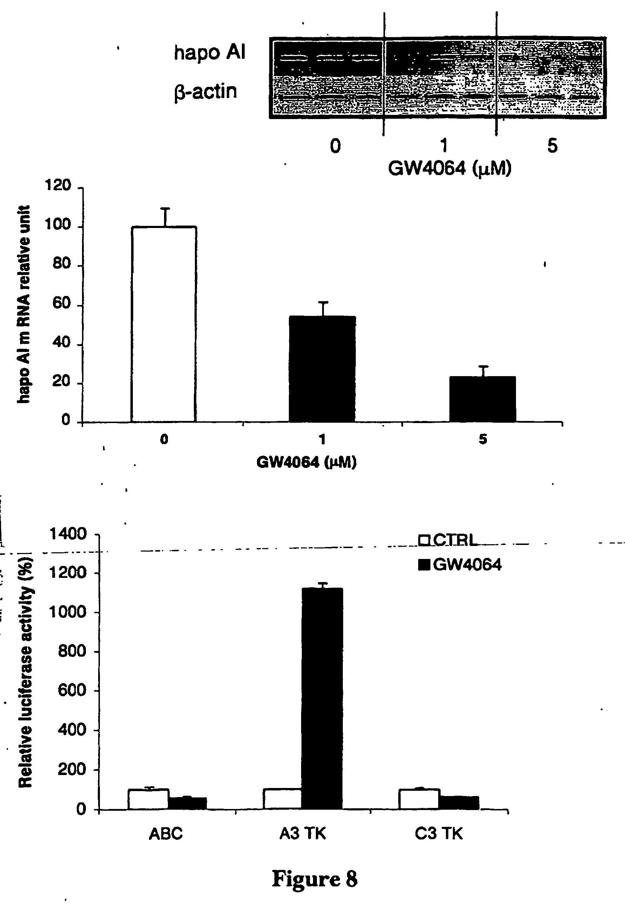

### Exemple 7 : Le GW4064, agoniste synthétique de FXR, réduit l'expression de l'apo AI dans les cellules HepG2 (figure 8)

Les cellules HepG2 sont co-transfectées comme précédemment décrit dans l'exemple 5. Les ARNm des cellules sont obtenus comme précédemment décrit dans l'exemple 2.

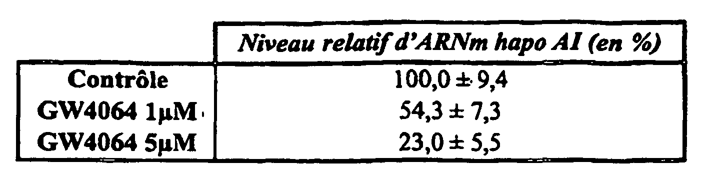

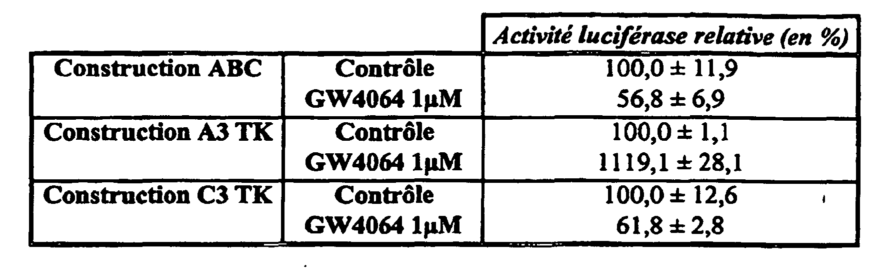

L'exemple 7 montre que le GW4064 (Goodwin et al., Mol Cell., 2000 Sep;6(3):517-26), puissant activateur spécifique de FXR, réduit fortement l'expression de l'apo AI dans les cellules HepG2.

L'exemple 7 montre que la sur-expression de hFXR en présence de 1µM de GW4064 réprime l'activité du fragment -254/+91 du promoteur de l'apo AI humaine (construction notée ABC) ainsi que celle de la construction qui comporte trois copies du site C (SEQ ID NO : 1) du promoteur de l'apo AI humaine (notée C3TK). On constate à l'inverse qu'une construction qui comporte trois copies du site A (SEQ ID NO : 2) du promoteur de l'apo AI humaine (notée A3TK) est activée par la sur-expression de hFXR en présence de GW4064.

## Revendications

1. Méthode pour la sélection, l'identification ou la caractérisation *in vitro* de composés capables de moduler le transport inverse du cholestérol, qui comprend :
- la mise en contact, en présence du récepteur FXR ou d'un équivalent fonctionnel du récepteur FXR, d'un composé test avec une construction d'acide nucléique comprenant au moins une copie d'un élément de réponse à FXR du promoteur du gène humain de l'apoliprotéine AI ou d'un variant fonctionnel de celui-ci, et
- la détermination de l'effet de la présence du composé test sur la liaison de FXR à l'élément de réponse.

2. Méthode selon la revendication 1, **caractérisée en ce que** l'élément de réponse à FXR comprend la séquence (SFQ. ID. NO: 1) suivante : ou un variant fonctionnel de celle-ci capable de lier le récepteur FXR.

3. Méthode selon la revendication 1, **caractérisée en ce que** l'élément de réponse à FXR comprend la séquence (SEQ. ID. NO: 2) suivante : ou un variant fonctionnel de celle-ci capable de lier le récepteur FXR.

4. Méthode pour la sélection, l'identification ou la caractérisation *in vitro* de composés capables de moduler le transport inverse du cholestérol, qui comprend :
- la mise en contact d'un composé test avec une cellule hôte comprenant le récepteur FXR ou un équivalent fonctionnel, un ligand de FXR et une cassette d'expression d'un gène rapporteur, ladite cassette comprenant un gène rapporteur placé sous le contrôle d'un promoteur consistant en au moins une copie d'un élément de réponse à FXR correspondant à la séquence (SEQ. ID.NO: 1) suivante : 5'-CAGAGCTGATCCTTGAACTCTTAAGTTCC-3' du promoteur du gène humain de l'apoliprotéine AI ou d'un variant fonctionnel de celui-ci, et
- la détermination de l'expression du gène rapporteur.

5. Méthode selon la revendication 4 comprenant la détermination du niveau d'expression du gène rapporteur en présence du composé test ou en l'absence dudit composé, une augmentation ou une diminution du niveau d'expression du gène rapporteur signalant l'aptitude du composé test à moduler le transport inverse du cholestérol.

6. Méthode selon la revendication 4 ou 5. **caractérisée en ce que** la cellule hôte est une cellule de mammifère.

7. Méthode selon la revendication 6, **caractérisée en ce que** la cellule de mammifère est une cellule humaine.

8. Méthode selon l'une des revendications 4 à 7, **caractérisée en ce que** le gène rapporteur est un gène codant un produit dont l'activité ou la présence dans des extraits biologiques peut être mesurée, notamment l'un des gènes codant pour la luciférase, la phosphatase alcaline sécrétée, la galactosidase ou la lactamase.

9. Méthode selon l'une des revendications 4 à 8, **caractérisée en ce que** le promoteur est choisi parmi le promoteur HSV-TK, le promoteur immédiat du CMV, le promoteur PGK et le promoteur du gène codant pour l'apo AI humaine, le promoteur SV40.

10. Méthode selon l'une des revendications 1 à 9. **caractérisée en ce qu'**un ou plusieurs composés sont testés, en mélange ou de manière séparée.

11. Méthode selon l'une des revendications 1 à 10, **caractérisée en ce que** le composé test est une banque combinatoire.

12. Méthode selon la revendication 11, **caractérisée en ce que** le composé test est un clone ou une banque de clones d'acides nucléiques exprimant un ou plusieurs polypeptide(s) liant l'ADN.

13. Méthode selon l'une quelconque des revendications précédentes, **caractérisée en ce que** la mise en contact est réalisée dans une plaque multipuits.

14. Méthode selon l'une quelconque des revendications précédentes comprenant, en outre, la comparaison des effets éventuels déterminés grâce à ladite méthode avec ceux, éventuels, déterminée grâce à une méthode réalisée dans les mêmes conditions mais avec une construction d'acide nucléique comprenant au moins une copie mutée de l'élément de réponse à FXR du promoteur du gène codant pour l'apo AI humain, ladite copie mutée étant essentiellement incapable de lier le récepteur FXR.

15. Méthode selon l'une quelconque des revendications précédentes, pour la sélection, l'identification ou la caractérisation de composés capables d'augmenter le transport inverse du cholestérol.

16. Méthode selon l'une quelconque des revendications 1 à 14. pour la sélection, l'identification ou la caractérisation de composés capables de moduler l'activité des HDL.

17. Méthode selon l'une quelconque des revendications 1 à 14, pour la sélection, l'identification ou la caractérisation de composés capables de moduler l'expression de l'apolipoprotéine AI.

## Patentansprüche

1. Verfahren zur *in vitro* Auswahl, Identifizierung oder Charakterisierung von Verbindungen, die in der Lage sind, den reversen Cholesterin-Transport zu modulieren, das umfasst:
- das Inkontaktbringen, in Anwesenheit des Rezeptors FXR oder eines funktionellen Äquivalentes des Rezeptors FXR, einer Testverbindung mit einem Nukleinsäure-Konstrukt, das wenigstens eine Kopie eines auf FXR antwortenden Elementes des Promotors des humanen AI-Apolipoprotein-Gens oder einer funktionellen Variante davon umfasst, und
- die Bestimmung der Wirkung der Anwesenheit der Testverbindung auf die Bindung von FXR an das antwortende Element.

2. Verfahren gemäß Anspruch 1, **dadurch gekennzeichnet, dass** das auf FXR antwortende Element die folgende Sequenz (SEQ. ID. NR: 1) umfasst: oder eine funktionelle Variante davon, die in der Lage ist, den Rezeptor FXR zu binden.

3. Verfahren gemäß Anspruch 1, **dadurch gekennzeichnet, dass** das auf FXR antwortende Element die folgende Sequenz (SEQ. ID. NR: 2) umfasst: oder eine funktionelle Variante davon, die in der Lage ist, den Rezeptor FXR zu binden.

4. Verfahren zur *in vitro* Auswahl, Identifizierung oder Charakterisierung von Verbindungen, die in der Lage sind, den reversen Cholesterin-Transport zu modulieren, das umfasst:
- das Inkontaktbringen einer Testverbindung mit einer Wirtszelle, die den Rezeptor FXR oder ein funktionelles Äquivalent, einen FXR-Liganden und eine Expressionskassette eines Reportergens umfasst, wobei besagte Kassette ein Reportergen umfasst, das unter der Kontrolle eines Promotors steht, der aus wenigstens einer Kopie eines auf FXR antwortenden Elementes besteht, das der folgenden Sequenz (SEQ. ID. NR: 1) entspricht: 5'-CAGAGCTGATCCTTGAACTCTTAAGTTCC-3' des Promotors des humanen AI-Apolipoprotein-Gens oder einer funktionellen Variante davon, und
- die Bestimmung der Expression des Reportergens.

5. Verfahren gemäß Anspruch 4, umfassend die Bestimmung des Expressionsniveaus des Reportergens in Anwesenheit der Testverbindung oder Abwesenheit besagter Verbindung, wobei eine Erhöhung oder Verringerung des Expressionsniveaus des Reportergens die Eignung der Testverbindung anzeigt, den reversen Cholesterin-Transport zu modulieren.

6. Verfahren gemäß Anspruch 4 oder 5, **dadurch gekennzeichnet, dass** die Wirtszelle eine Säugerzelle ist.

7. Verfahren gemäß Anspruch 6, **dadurch gekennzeichnet, dass** die Säugerzelle eine humane Zelle ist.

8. Verfahren gemäß einem der Ansprüche 4 bis 7, **dadurch gekennzeichnet, dass** das Reportergen ein Gen ist, das für ein Produkt codiert, dessen Aktivität oder Vorhandensein in biologischen Extrakten gemessen werden kann, insbesondere eines der Gene, die für die Luciferase, sezernierte alkalische Phosphatase, Galactosidase oder Lactamase codieren.

9. Verfahren gemäß einem der Ansprüche 4 bis 8, **dadurch gekennzeichnet, dass** der Promotor aus dem HSV-TK-Promotor, dem unmittelbar frühen Promotor des CMV, dem PGK-Promotor und dem Promotor des Gens, das für humanes AI-Apo codiert, dem SV40-Promotor ausgewählt ist.

10. Verfahren gemäß einem der Ansprüche 1 bis 9, **dadurch gekennzeichnet, dass** eine oder mehrere Verbindungen als Gemisch oder getrennt getestet werden.

11. Verfahren gemäß einem der Ansprüche 1 bis 10, **dadurch gekennzeichnet, dass** die Testverbindung eine kombinatorische Bank ist.

12. Verfahren gemäß Anspruch 11, **dadurch gekennzeichnet, dass** die Testverbindung ein Nukleinsäure-Klon oder eine Bank von Nukleinsäure-Klonen ist, der/die ein oder mehrere DNA-bindende Polypeptide exprimiert/exprimieren.

13. Verfahren gemäß einem der vorhergehenden Ansprüche, **dadurch gekennzeichnet, dass** das Inkontaktbringen auf einer Mikrotiterplatte durchgeführt wird.

14. Verfahren gemäß einem der vorhergehenden Ansprüche, außerdem umfassend den Vergleich möglicher Wirkungen, die mit Hilfe besagten Verfahrens bestimmt sind, mit denjenigen möglichen, die mit Hilfe eines Verfahrens bestimmt sind, das unter denselben Bedingungen aber mit einem Nukleinsäure-Konstrukt durchgeführt ist, das wenigstens eine mutierte Kopie des auf FXR antwortenden Elementes des Promotors des Gens umfasst, das für das humane AI-Apo codiert, wobei besagte mutierte Kopie im Wesentlichen nicht in der Lage ist, den Rezeptor FXR zu binden.

15. Verfahren gemäß einem der vorhergehenden Ansprüche zur Auswahl, Identifizierung oder Charakterisierung von Verbindungen, die in der Lage sind, den reversen Cholesterin-Transport zu erhöhen.

16. Verfahren gemäß einem der Ansprüche 1 bis 14 zur Auswahl, Identifizierung oder Charakterisierung von Verbindungen, die in der Lage sind, die HDL-Aktivität zu modulieren.

17. Verfahren gemäß einem der Ansprüche 1 bis 14 zur Auswahl, Identifizierung oder Charakterisierung von Verbindungen, die in der Lage sind, die Expression des AI-Apolipoproteins zu modulieren.

## Claims

1. Method for *in vitro* selecting, identifying or characterizing compounds capable of modulating reverse cholesterol transport, which comprises :
- contacting, in the presence of the FXR receptor or a functional equivalent of the FXR receptor, a test compound with a nucleic acid construct comprising at least one copy of an FXR response element of the human apolipoprotein A-I gene promoter or a functional variant thereof, and
- determining the effect of the presence of the test compound on the binding of FXR to the response element.

2. Method according to claim 1, wherein the FXR response element comprises the following sequence (SEQ ID NO : 1) : or a functional variant thereof capable of binding the FXR receptor.

3. Method according to claim 1, wherein the FXR response element comprises the following sequence (SEQ ID NO : 2) : or a functional variant thereof capable of binding the FXR receptor.

4. Method for *in vitro* selecting, identifying or characterizing compounds capable of modulating reverse cholesterol transport, which comprises :
- contacting a test compound with a host cell comprising the FXR receptor or functional equivalent thereof, an FXR ligand, and an expression cassette of a reporter gene, said cassette comprising a reporter gene placed under the control of a promoter consisting of at least one copy of an FXR response element corresponding to the following sequence (SEQ ID NO : 1) : 5'-CAGAGCTGATCCTTGAACTCTTAAGTTCC-3', of the human apolipoprotein A-I gene promoter or a functional variant thereof, and
- determining the expression of the reporter gene.

5. Method according to claim 4, comprising determining the level of reporter gene expression in the presence of the test compound or in the absence of said compound, an increase or a decrease in the level of reporter gene expression indicating the capacity of the test compound to modulate reverse cholesterol transport.

6. Method according to claim 4 or 5, wherein the host cell is a mammalian cell.

7. Method according to claim 6, wherein the mammalian cell is a human cell.

8. Method according to any one of claims 4 to 7, wherein the reporter gene is a gene coding for a product whose activity or presence in biological extracts can be measured, particularly one of the genes coding for luciferase, secreted alkaline phosphatase, galactosidase or lactamase.

9. Method according to any one of claims 4 to 8, wherein the promoter is selected from the HSV-TK promoter, the immediate early CMV promoter, the PGK promoter and the promoter of the gene encoding human apolipoprotein A-I, the SV40 promoter.

10. Method according to any one of claims 1 to 9, wherein one or several compounds are tested, as a mixture or separately.

11. Method according to any one of claims 1 to 10, wherein the test compound is a combinatorial library.

12. Method according to claim 11, wherein the test compound is a clone or a library of nucleic acid clones expressing one or several DNA=binding polypeptide(s).

13. Method according to any one of the preceding claims, wherein the placing in contact is carried out in a multiwell plate.

14. Method according to any one of the preceding claims comprising, additionally, comparing the possible effects determined by said method with the possible effects determined by a method carried out in the same conditions but with a nucleic acid construct comprising at least one mutated copy of the FXR response element of the human apolipoprotein A-I gene promoter, said mutated copy being substantially incapable of binding the FXR receptor.

15. Method according to any one of the preceding claims, for selecting, identifying or characterizing compounds capable of increasing reverse cholesterol transport.

16. Method according to any one of claims 1 to 14, for selecting, identifying or characterizing compounds capable of modulating HDL activity.

17. Method according to any one of claims 1 to 14, for selecting, identifying or characterizing compounds capable of modulating the expression of apolipoprotein A-I.


### LISTE DE SEQUENCES

<110> GENFIT SA
<120> PROCEDES D'IDENTIFICATION DE COMPOSES MODULANT LE TRANSPORT INVERSE DU CHOLESTEROL
<130> B0050WO
<140>
   <141>
<160> 6
<170> PatentIn Ver. 2.1
<210> 1
   <211> 29
   <212> ADN
   <213> Séquence artificielle
<220>
   <223> Description de la séquence artificielle: Site C wt
<400> 1
   cagagctgat ccttgaactc ttaagttcc 29
<210> 2
   <211> 33
   <212> ADN
   <213> Séquence artificielle
<220>
   <223> Description de la séquence artificielle: Site A wt
<400> 2
   ccccactgaa cccttgaccc ctgccctgca gcc 33
<210> 3
   <211> 29
   <212> ADN
   <213> Séquence artificielle
<220>
   <223> Description de la séquence artificielle: Site C mt
<400> 3
   cagagctata tattatatat ataagttcc 29
<210> 4
   <211> 33 4
   <212> ADN
   <213> Séquence artificielle
<220>
   <223> Description de la séquence artificielle: Site A mt
<400> 4
   ccccactgaa cccttgattc ctgctttgca gcc 33
<210> 5
   <211> 349
   <212> ADN
   <213> Séquence artificielle
<220>
   <223> Description de la séquence artificielle: Promoteur ApoAI
<400> 5
   gggagacctg caagcctgca gcactcccct cccgccccca ctgaaccctt gacccctgcc 60
   ctgcagcccc cgcagcttgc tgtttgccca ctctatttgc ccagccccag ggacagagct 120
   gatccttgaa ctcttaagtt ccacattgcc aggaccagtg agcagcaaca gggccggggc 180
   tgggcttatc agcctcccag cccagaccct ggctgcagac ataaataggc cctgcaagag 240
   ctggctgctt agagactgcg agaaggaggt gcgtcctgct gcctgccccg gtcactctgg 300
   ctccccagct caaggttcag gccttgcccc aggccgggcc tctgggtac 349
<210> 6
   <211> 166
   <212> ADN
   <213> Séquence artificielle
<220>
   <223> Description de la séquence artificielle: Promoteur tk
<400> 6
   tgccccgccc agcgtcttgt cattggcgaa ttcgaacacg cagatgcagt cggggcggcg 60
   cggtccaggt ccacttcgca tattaaggtg acgcgtgtgg cctcgaacac cgagcgaccc 120
   tgcagcgacc cgcttaacag cgtcaacacg tgccgcagat cacgag 166